# EUROPEAN PATENT APPLICATION

(11) **EP 4 302 678 A1**
(43) Date of publication of application: **10.01.2024**
(21) Application number: 22187280.7
(22) Date of filing: 27.07.2022
(51) Int. Cl.: A61B 1/005

(54) **BENDABLE TUBE FOR USE IN AN ENDOSCOPE**

(30) Priority: 07.07.2022 EP 22183657
(71) Applicant: HOYA CORPORATION, Shinjuku-ku Tokyo 160-8347 (JP)
(72) Inventor: Walberg, Erik, 86316 Friedberg (DE)
(74) Representative: Lucke, Andreas

(57) **Abstract**

Disclosed herein is a tube for use in an endoscope. The tube comprises a plurality of elements that are arranged along a center line of said tube. The elements are pivotably connected to each other by a plurality of joints. Said joints allow for bending the tube with respect to a default configuration of the tube. Each of the joints comprises a joint head on a respective first element of the plurality of elements and a joint socket on a respective second element of the plurality of elements, wherein the joint socket is configured to receive the joint head. The joint socket and the joint head are shaped such that, when the first element is pivoted with respect to the second element from a default articulation angle of the joint associated with said default configuration to a target articulation angle, a first contact surface on the joint head engages a second contact surface on the joint socket to cause the joint head to be pushed outwards out of the joint socket such that a displacement of the joint head along said center line of the tube relative to its position at the default articulation angle is greater than zero for any target articulation angle.

## Description

### FIELD OF THE DISCLOSURE

The present disclosure is in the field of medical technology. In particular, the present disclosure relates to a bendable tube for use in an endoscope that includes a plurality of elements pivotably connected to each other by a plurality of joints.

### BACKGROUND

An endoscope is a surgical device that may be used to access (e.g., view or remove) or treat tissue within the body of a patient by inserting one or more medical tools into the body through an incision in the body or an orifice of the body. The endoscope may include (comprise) an interface/control portion and an insertion tube that is coupled to the interface/control portion. The insertion tube is configured to be inserted into the body of the patient and may include one or more channels to provide access to tissue within the body. The one or more channels may e.g. be configured to receive a medical tool and/or a fluid and to guide the medical tool and fluid, respectively, to the tissue of interest.

The insertion tube may be bendable to facilitate insertion into the patient's body. For this, the insertion tube may for example include one or more passively and/or actively bendable sections (also referred to as passive and active bending section, respectively). A passively bendable section may e.g. be formed of a bendable or flexible material or structure that can be deformed by applying an external force. An actively bendable section may include a plurality of joints that can be articulated, e.g. for actively navigating a tip of the endoscope at a distal end of the insertion tube. Each of the joints may for example include a circular joint head arranged in a circular joint socket such that the joint head can pivot with respect to the joint socket, thereby allowing for bending the insertion tube.

Such actively bendable sections may, however, be mechanically unstable as the joints may have a tendency to align in a zig-zag pattern with adjacent joints being tilted in opposite directions. This may not only lead to a decrease in stability against side loads, but may also reduce the effective inner cross-sectional area of the insertion tube. Available space inside the tube may be reduced further due to the need for arranging means for controlling the joints such as control wires. Furthermore, a clearance between the joint head and socket may be required to prevent the joint from jamming. This may result in less efficient propagation of torque along the tube since the clearance may first have to be compensated before torque is transferred by the joint.

### SUMMARY OF THE DISCLOSURE

It is thus a feature of the present disclosure to provide a tube for use in an endoscope that exhibits improved mechanical stability and efficient torque transfer while also providing sufficient space inside the tube.

The present disclosure provides a tube for use in an endoscope as set out in the appended independent claim. Examples thereof are detailed in the dependent claims.

The tube according to the present disclosure is configured for use in an endoscope. The tube includes (i.e. including, but not limited to) a plurality of elements that are arranged along a center line of said tube. The elements are pivotably connected to each other by a plurality of joints that allow for bending the tube with respect to a default configuration of the tube. Each of the joints includes a joint head on a respective first element of the plurality of elements and a joint socket on a respective second element of the plurality of elements, wherein the joint socket is configured to receive the joint head. The joint socket and the joint head are shaped such that, when the first element is pivoted with respect to the second element from a default articulation angle of the joint associated with said default configuration to a target articulation angle, a first contact surface on the joint head engages a second contact surface on the joint socket to cause the joint head to be pushed outwards out of the joint socket such that a displacement of the joint head along said center line of the tube relative to its position at the default articulation angle is greater than zero for any target articulation angle.

The tube may extend from a proximal end of the tube to a distal end. As used herein, the term "proximal" may refer to an element, a feature or a location along the length of the tube that is to be closer to a physician or other medical practitioner during use of the endoscope (e.g. closer to an interface/control portion of the endoscope), whereas the term "distal" may refer to an element, a feature or a location along the length of the tube that is to be closer to a location of tissue being examined or treated within the body of a patient during use of the endoscope (e.g. further away from an interface/control portion of the endoscope). A direction along the center line of the tube (e.g. from the proximal end to the distal end) may also be referred to as a longitudinal or axial direction in the following. A direction along a circumference of the tube (for example parallel to a surface, e.g. an outer surface, of the tube and perpendicular to the longitudinal direction) may also be referred to as a circumferential or azimuthal direction. A direction perpendicular to a surface, e.g. an outer surface, of the tube (for example perpendicular to the longitudinal direction and to the circumferential direction) may also be referred to as a radial direction. The longitudinal, circumferential and radial directions may for example define a cylindrical coordinate system. Depending on the state or configuration of the tube, the orientation of these directions may vary along the length of the tube, e.g. as a result of a bending of the tube.

The elements (also referred to as tube elements in the following) may for example be annular or tubular elements. The elements may e.g. have channel openings, which together may form a channel (or lumen) of the tube when the elements are connected by the joints. In some examples, some or all of the elements may have the same shape and/or physical dimensions and may in particular be identical elements (e.g. elements made from the same material and having the same shape and physical dimensions). The tube elements are arranged along the longitudinal direction, e.g. in a chain as detailed below. The tube elements may be arranged in one or more sections along a length of the tube (i.e. between the proximal and distal ends of the tube), e.g. in an active bending section at or adjacent to a distal end of the tube. In some examples, some or all of the tube elements may be directly connected to each other via the joints, i.e. without any additional members or elements therebetween. In other examples, one or more additional elements or members of the tube may be arranged between adjacent tube elements, wherein the additional elements or members may e.g. be rigidly connected between adjacent tube elements and/or may be pivotably connected between adjacent tube elements by joints or hinges different from said joints according to the present disclosure (e.g. by joints or hinges having a circular joint head and a circular joint socket).

The center line of the tube may extend along a center of the tube or along a center of a channel of or arranged in the tube. The center line may for example be a line or path connecting the centers (e.g. the centroids) of the outer or inner circumferences of the tube along the length of the tube. Depending on the state or configuration of the tube, the center line of the tube may not be a straight line, but may e.g. follow a curved path.

The tube elements are pivotably connected to each other by the plurality of joints such that adjacent elements can be pivoted (e.g. tilted or rotated) with respect to each other. Thereby, the tube can be bent (or deformed) with respect to its default configuration. The default configuration may for example be the configuration or state that the tube ideally assumes when a uniform tension or force (e.g. without any bias in a certain direction) is applied along the length of the tube, e.g. between the proximal and distal ends of the tube. In some examples, the default configuration may be the configuration or state that the tube ideally assumes when no external tension or force is applied to the tube (e.g. when not actuating means for actuating the joints such as control wires). The default configuration may be associated with a predefined bending radius that the tube or a part thereof assumes when the tube is in the default configuration. The default configuration may in particular by a straight configuration (corresponding to an infinite bending radius), i.e. the tube may be straight or substantially straight in the default configuration.

Each of the joints is configured to pivotably connect a respective first element (e.g. a respective proximal or distal element) to a respective second element (e.g. a respective distal and proximal element, respectively). For this, the joint head on the respective first element may be received in the joint socket on the respective second element at least in part. The joint head may for example be a protrusion or projection on the first element, which may e.g. extend along the longitudinal direction. The joint socket may for example be a corresponding cut-out or recess on the second element that the joint head or a part thereof can be arranged in. The joint socket and the joint head include a first and second contact surface, respectively, that are configured to engage each other (e.g. to come in contact with each other) so as to permit articulation of the joint, e.g. by moving or sliding the first contact surface along the second contact surface.

In the default configuration, each joint assumes a respective default articulation angle, in some examples a same default articulation angle (i.e. the default articulation angle of some or all of the joints may be equal and may e.g. be 0°). The articulation angle may for example be the angle at which the first and second elements are arranged with respect to each other (e.g. the angle by which the first element is tilted with respect to the second element). A default articulation angle of 0° may e.g. correspond to a configuration in which the respective first and second elements are straight or parallel to each other.

The joint socket and head of each of the joints are configured such that when the first element is pivoted with respect to the second element away from the default articulation angle of the respective joint, the joint head is pushed out of the joint socket (e.g. along the longitudinal direction/center line) at least in part. In other words, the motion of the joint head not only constitutes a rotation about a stationary rotation or articulation axis (i.e. cannot be decomposed into a pure rotation about a same rotation axis for all articulation angles), but additionally includes a translation outwards out of the joint socket.

Thereby, the joint head - as seen from the joint socket (e.g. in the reference frame of the joint socket) - is displaced (e.g. along the center line) from its default position at the default articulation angle of the respective joint. This may cause the first element to move or be pushed away from the second element (e.g. cause the first and second elements to be separated or separated further from each other) such that the joint and thereby the tube extends slightly in the longitudinal direction. Put differently, the joint socket and head are configured such that the length of the tube (e.g. the length of the center line between the proximal and distal ends) increases when the joint is pivoted away from its default articulation angle.

The joint socket and head are shaped such that the displacement of the joint head along the center line relative to its position at the default articulation angle is greater than zero at any target articulation angle (e.g. at any articulation angle other than the default articulation angle). In other words, the displacement of the joint head (and hence the length of the tube) exhibits a global minimum at the default articulation angle. This may bias the tube towards the default configuration, for example such that the tube preferably assumes the default configuration, e.g. when the tube is under uniform longitudinal tension. The displacement of the joint head along the center line may for example be measured by projecting a line connecting corresponding points on the joint head at the default articulation angle and at the respective target articulation angle onto the center line. Said points on the joint head may e.g. be a centroid or a center of mass of the joint head or a point on the joint head through which an articulation axis of the joint extends at the default articulation. The displacement may for example increase by between 0.1 µm and 0.1 mm, in some examples by between 0.5 µm and 50 µm, in one example by between 1 µm and 10 µm per degree (°) of articulation away from the default articulation angle. The displacement at a maximum articulation angle of the joint (e.g. at one or both ends of an operable range of the joint) may for example be between 2 µm and 1 mm, in some examples between 5 µm and 0.5 mm, in one example between 20 µm and 0.2 mm and in one example between 0.10 mm and 0.15 mm.

In some examples, the first contact surface includes a protruding portion that protrudes from an inscribed circle of the first contact surface and/or the second contact surface includes a recessed portion that is recessed from an inscribed circle of the second contact surface. Put differently, one or both of the joint head and the joint socket may be non-circular, i.e. at least portions thereof may deviate from a circular shape or cross-section (e.g. when seen perpendicular to the longitudinal direction), wherein the joint head includes a protrusion as compared to a circular shape and the joint socket includes a recess as compared to a circular shape. The protruding portion may for example protrude from the inscribed circle of the first contact surface by between 2% and 30%, in some examples by between 4% and 20%, in one example by between 6% and 15% of a radius of the inscribed circle of the first contact surface. Additionally or alternatively, the protruding portion may for example protrude from the inscribed circle of the first contact surface by between 5 µm and 0.5 mm, in some examples by between 20 µm and 0.2 mm and in one example by between 0.10 mm and 0.15 mm. The recessed portion may for example be recessed from the inscribed circle of the second contact surface by between 3% and 50%, in some examples by between 5% and 30%, in one example by between 10% and 20% of a radius of the inscribed circle of the second contact surface. Additionally or alternatively, the recessed portion may for example be recessed from the inscribed circle of the second contact surface by between 10 µm and 1 mm, in some examples by between 30 µm and 0.3 mm and in one example by between 0.15 mm and 0.2 mm.

The inscribed circle of a contact surface used herein may for example be a circle to which at least two non-adjacent portions of the respective contact surface (e.g. portions on opposite sides of the joint head and socket, respectively) are tangential. The inscribed circle may be in a plane perpendicular to the radial direction of the tube and/or in plane perpendicular to an articulation axis of the joint. In some examples, said at least two non-adjacent portions may be portions of the respective contact surface that are in contact with the other contact surface when the first element is at the default articulation angle. The inscribed circle may for example connect points on said portions of the respective contact surface, in particular the centers of said portions of the respective contact surface. In one example, the inscribed circle may be the largest circle to which at least two non-adjacent portions of the respective contact surface are tangential, wherein said two non-adjacent portions are in contact with the other contact surface when the first element is at the default articulation angle.

The protruding portion and the recessed portion may be arranged on the respective contact surface such that the protruding portion of the first contact surface faces the recessed portion of the second contact surface when the first element is at the default articulation angle. In other words, when the first element is at the default articulation angle, the protrusion on the joint head may be arranged in the recess in the joint socket. This may allow for arranging the joint head further within the joint head than at other articulation angles.

When the first element is pivoted from the default articulation angle to the target articulation angle, the protruding portion may engage the second contact surface to cause the joint head to be pushed outwards out of the joint socket. For example, the protruding portion may come in contact with the second contact surface (e.g. a non-recessed portion thereof) and thereby may force the joint head further out of the joint socket. Put differently, the non-circular shape of the joint head and joint socket may prevent pivoting or rotation of the joint about a stationary articulation axis and may additionally induce a translation of the joint head relative to the joint socket as described above.

In some examples, the first contact surface may include a first straight portion and a second straight portion extending at an angle to the first straight portion. Additionally or alternative, the second contact surface may include a third straight portion and a fourth straight portion. As used herein, a straight portion of a contact surface may for example be a portion having one or both principal curvatures equal to zero (i.e. portions that exhibit no curvature in at least one direction, e.g. concave/convex ellipsoid portions or planar portions). For example, one or both of the joint head and the joint socket may have a triangular or V-shape or cross-section with a pair of straight edges or surfaces extending at an angle to each other (e.g. when seen along an articulation axis of the joint and/or along the radial direction). The first and second straight portions of the first contact surfaces may be arranged such that, when the first element is at the default articulation angle, the first and second straight portions each are in contact with a respective straight portion (e.g. the third or fourth straight portion) of the second contact surface. This may e.g. allow for establishing a line contact or a planar contact rather than a point contact between the first and second contact surfaces at the default articulation angle. This may provide a robust and well-defined contact, in particular when taking into account manufacturing tolerances.

The protruding portion of the first contact surface may be arranged between the first and second straight portions of the first contact surface. In some examples, the first and second straight portions of the first contact surface maybe tangential to the inscribed circle of the first contact surface. Additionally or alternatively, the recessed portion of the second contact surface may be arranged between the third and fourth straight portions of the second contact surface. In some examples, the third and fourth straight portions of the second contact surface may be tangential to the inscribed circle of the second contact surface. For example, one or both of the joint head and the joint socket may have a flat-topped triangular or V-shape or cross-section with a flat or rounded tip. The flat or rounded tip may form the protruding and recessed portion, respectively, of the corresponding contact surface.

In some examples, the joint socket and the joint head are shaped such that the displacement of the joint head along the center line of the tube relative to its position at the default articulation angle increases monotonically, in one example strictly monotonically, when the first element is pivoted from the default articulation angle to a target articulation angle that differs from the default articulation angle by no less than 10°, in some examples by no less than 20°, in one example by no less than 30°. The displacement may for example increase monotonically, in one example strictly monotonically, up to a target articulation angle that differs from the default articulation angle by between 10° and 75°, in some examples by between 20° and 60°, in one example by between 30° and 50°. Additionally or alternatively, said displacement of the joint head may increase monotonically, in one example strictly monotonically when the first element is pivoted from the default articulation angle to a maximum articulation angle of the joint (e.g. the displacement may increase monotonically or strictly monotonically over the entire operable range of the joint). Put differently, at any articulation angle, the displacement of the joint head (and thus the length of the tube) may decrease when pivoting the first element towards the default articulation angle while the displacement may increase when pivoting the first element away from the default articulation angle towards the maximum articulation angle.

In some examples, the joint socket and the joint head are shaped such that a tip of the joint head is separated from the joint socket by a gap when the first element is at the default articulation angle. For example, the protruding portion of the first contact surface and the recessed portion of the second contact surface may be shaped such that the protruding portion is separated from the recessed portion by a gap when the first element is at the default articulation angle. The gap may for example be between 5 µm and 0.5 mm, in some examples between 10 µm and 0.1 mm, in one example between 40 µm and 60 µm.

In some examples, the joint socket may be configured such that the joint head can be removed from the joint socket along the center line of the tube. For example, a width of an opening of the joint socket may be larger than a maximum width of the joint head (e.g. along the circumferential direction) such that the joint head can freely be moved into and out of the joint socket through said opening. Such an open joint geometry, in which the joint head is not confined to the joint socket, may for example allow the joint head to leave the joint socket when excessive force is applied to the tube and may thus prevent damage to the joint in some examples. During normal use (e.g. without applying excessive force), the join head may for example be held in the joint socket by means for actuating the joint such as control wires, which may for example be configured to apply a uniform compressive tension in the longitudinal direction when not being actuated.

In some examples, the joint socket and/or the joint head include a lateral mechanical stop configured to limit a movement of the joint head relative to the joint socket in a direction parallel to an articulation axis of the joint (e.g. in the radial direction). The lateral mechanical stop may for example be or include one or more lateral sidewalls of the joint socket, which may e.g. be arranged at an inner circumference and/or an outer circumference of the respective element (e.g. an inner lateral sidewall and/or an outer later sidewall of the joint socket). The one or more lateral sidewalls may be configured to come in contact with the joint head when the joint head is moved parallel to the articulation axis to prevent the joint head from leaving the joint socket laterally. Additionally or alternatively, a lateral mechanical stop may also be provided on the joint head, e.g. a lateral mechanical stop extending from the joint head along an inner and/or outer sideface of the adjacent element including the corresponding joint socket.

In some examples, one or more joints of the plurality of joints may have a maximum articulation angle that is different from a maximum articulation angle of the other joints of the plurality of joints. This may for example allow for implementing non-uniform bending profiles, e.g. bending profiles having a non-uniform bending radius (i.e. deviating from a circular shape). For example, the plurality of joints may include two or more groups of joints, wherein each of the groups may have a respective maximum articulation angle (which may e.g. be associated with a respective minimum bending radius). The plurality of joints may for example include a first group of joints (including one or more joints) having a first maximum articulation angle and a second group of joints (including one or more joints, e.g. all of the joints not included in the first group) having a second maximum articulation angle different from the first maximum articulation angle. The first maximum articulation angle may for example be between 1.25 times and 10 times, in some examples between 1.5 times and 3 times the second maximum articulation angle or vice-versa. The first and second maximum articulation angles may for example differ by between 2° and 30°, in one example by between 5° and 20° from each other. In some examples, the maximum articulation angle of the joints may vary continuously along the length of the tube. The maximum articulation angle may for example increase (or decrease) monotonically, in one example strictly monotonically along the length of the tube, e.g. such that the maximum articulation angle of any given joint is larger than the maximum articulation angle of a proximally (or distally) adjacent joint. Different maximum articulation angles may for example be achieved by providing angled end faces on the elements that extend at different angles as detailed below. In other examples, all joints may have the same maximum articulation angle.

In some examples, the tube elements are arranged in a chain, e.g. a serial arrangement in which each of the tube elements (apart from the outermost elements) is connected to a respective proximal element by a respective proximal joint and to a respective distal element by a respective distal joint. In some examples, an outermost joint at one or both ends of the chain may have a smaller maximum articulation angle than the other joints in the chain. For example, the maximum articulation angle of the outermost joint at one or both ends of the chain may be between 10% and 70%, in some examples between 20% and 50%, in one example between 25% and 45% (e.g. 1/3) and in one example between 50% and 70% (e.g. 2/3) of the maximum articulation angle of the other joints in the chain. In some examples, the other joints in the chain all have the same maximum articulation angle.

For each of the joints, the first element may include a first angled end face and the second element may include a second angled end face facing the first angled end face. The first and second end faces may be configured to come in contact with each other when the joint reaches its maximum articulation angle to prevent further articulation of the joint (e.g. serve as an axial or longitudinal mechanical stop). When the joint is not at the maximum articulation angle, the first and second end faces may be spaced apart from each other to permit articulation of the joint. In the default configuration, the first and second end faces may each extend at a respective angle relative to a plane perpendicular to the center line of the tube. The first and second angled end faces may be angled in opposite directions, e.g. such that the sign of the angle of the first angled end face is opposite to the sign of the angle of the second angle end face. In some examples, a respective contact feature such as a protrusion or projection may be arranged on or formed by the first end face and/or the second end face. Said contact feature may be configured to come in contact with the other end face, e.g. a contact feature on the other end face, when the joint reaches its maximum articulation angle, e.g. to provide a well-defined contact point.

An angle between the first and second end faces associated with the outermost joint at one or both ends of the chain may be smaller than an angle between the first and second end faces associated with the other joints in the chain when the respective joints are at the default articulation angle. The angle between the first and second angled end faces in the default configuration may define (e.g. correspond to) the maximum articulation angle. The angle between the first and second end faces associated with the other joints in the chain may for example be between 25° and 55°, in one example between 35° and 45°. The angle between the first and second end faces associated with the outermost joint at one or both ends of the chain may for example be between 5° and 45°, in one example between 5° and 20° (e.g. 1/3 of the angle between the first and second end faces associated with the other joints) and in one example between 25° and 35° (e.g. 2/3 of the angle between the first and second end faces associated with the other joints).

In some examples, some or all of the tube elements include a guiding element that is configured to guide a control wire for actuating the plurality of joints. The guiding element may for example be or include a groove, a notch, a clip, a hook and/or a hole in which the control wire can be arranged.

In some examples, the tube may also include the control wire, wherein the control wire may be arranged in said guiding elements of the tube elements. The control wire may for example be configured to, when actuated, apply a compressive tension to the tube or a part thereof, in particular a section including the tube elements, along the longitudinal direction, e.g. from the distal end to the proximal end. The control wire may for example be connected or attached to a distal attachment point, which may for example be arranged on a distal side of the tube elements, e.g. at or adjacent to the distal end or tip of the tube. When pulling on a proximal end of the control wire, a force may be generated from the distal attachment point towards the proximal end of the tube. In some examples, the control wire may also be configured to apply a compressive tension even when not actuated, for example to bias the tube towards the default configuration and/or to secure the joint heads in the joint sockets.

The guiding element may for example be arranged at an outer circumference of the respective element, e.g. on an outer surface of the element that faces away from a central opening of the element (i.e. facing radially outwards as seen from the center line of the tube). The guiding element may in particular be arranged such that the control wire, when arranged in the guiding element, is located on the outside of tube, i.e. not in or facing the central opening. This may for example increase the available space inside the tube.

In one example, the guiding element is configured to guide the control wire such that the control wire is arranged adjacent to an inner or outer sideface of a joint head on the respective element or on an adjacent element. The guiding element may for example be aligned with the joint head or a joint socket on the respective element along the circumferential direction, e.g. such that the control wire, when arranged in the guiding element, extends adjacent to the respective joint, for example through or adjacent to an actuation axis of the joint. The control wire arranged adjacent to the inner or outer sideface of a joint head may for example provide a lateral mechanical stop for the respective joint head, e.g. to prevent said joint head from leaving the joint socket in the radial direction.

In some examples, some or all of the elements each are pivotably connected to a respective first adjacent element (e.g. a respective proximal element) by a respective first pair of joints arranged on opposite sides of the tube. The joints of the first pair may for example be displaced by between 150° and 210°, in one example by between 170° and 190° with respect to each other along the circumferential direction. In some examples, some or all of the first pairs of joints may be arranged in a same plane, e.g. to allow for bending the tube in a first articulation direction or first articulation plane.

Additionally or alternatively, some or all of the elements (in particular some or all of said elements pivotably connected to a respective first adjacent element by a respective first pair of joints) each are pivotably connected to a respective second adjacent element (e.g. a respective distal element) by a respective second pair of joints arranged on opposite sides of the tube. The joints of the second pair may for example be displaced by between 150° and 210°, in one example by between 170° and 190° with respect to each other along the circumferential direction. In some examples, some or all of the second pairs of joints may be arranged in a same plane, e.g. to allow for bending the tube in a second articulation direction or second articulation plane.

The first and second pairs of joints may be displaced with respect to each other by between 75° and 105°, in some examples by between 80° and 100°, in one example by between 85° and 95° (e.g. by 90°) along the circumferential direction of the tube. In other words, on each of the corresponding tube elements, the joint heads and/or sockets associated with the respective first and second pairs of joints may be arranged alternatingly and equidistantly or substantially equidistantly along the circumferential direction, e.g. displaced by 90° from adjacent joint heads and/or sockets. Accordingly, the first and second articulation directions/planes may be orthogonal or substantially orthogonal to each other.

Some or all of said tube elements that are pivotably connected to respective adjacent elements by the first and second pairs of joints may each include four guiding elements arranged along a circumference of the respective element, each of said four guiding elements being configured to guide a respective control wire. Each of said four guiding elements may be displaced by between 35° and 55°, in some examples by between 40° and 50° (e.g. 45°) from adjacent joints along the circumferential direction of the tube. Put differently, each of said guiding elements maybe arranged about halfway between two of the joints associated with the respective tube element (i.e. between a joint of the respective first pair and a joint of the respective second pair). In some examples, two of said guiding elements may be arranged in a first wire plane and the other two guiding elements may be arranged in a second wire plane. The first and second wire planes may be at an angle between 35° and 55°, in some examples by between 40° and 50° (e.g. 45°) to the first and second articulation planes. As the wire planes are not aligned with the articulation directions/planes, actuation of each of the control wires may actuate all of the joints simultaneously. This may e.g. result in a bending of the tube in the respective wire plane.

In other examples, each of the four guiding elements may be aligned with a corresponding joint along the circumferential direction, e.g. such that the wire planes are aligned with the articulation directions/planes. In this case, actuation of each of the control wires may only actuate either the first pairs or the second pairs of joints and may result in a bending of the tube in the respective articulation direction/plane.

Each of the tube elements may extend around a respective channel opening as mentioned above, e.g. such that a sidewall of each tube elements encloses or surrounds the respective channel opening along the circumferential direction. The channel openings may have a circular or substantially circular cross-section perpendicular to the center line of the tube. For example, a diameter of the channel openings may vary by less than 25% (e.g. by between 0% and 25%, in some examples by between 5% and 25%, in one example by between 10% and 25%), preferably by less than 15%, in one example by less than 10% along the circumference of the respective element. The variation of the diameter of the channel opening may for example be defined as a ratio of a difference between a maximum diameter and a minimum diameter to an average diameter of the channel opening. The tube elements may have a smooth inner surface along their inner circumference. For example, a minimum radius of curvature of the inner surface in a plane perpendicular to the center line may be no less than 10% (e.g. between 10% and 100%), in some examples no less than 20% (e.g. between 20% and 100%), in one example no less than 30% (e.g. between 30% and 100%) and in one example no less than 40% (e.g. between 40% and 100%) of a mean radius of the channel opening.

The tube according to the present disclosure is configured for use in an endoscope. The tube may, for example and without limitation, be configured for use in a bronchoscope, a sinuscope, a nasopharyngoscope, a laryngoscope, a laparoscope, a gastroscope, a duodenoscope, a colonoscope, an echoendoscope, a hysteroscope, a cystoscope, a uroscope, a urethroscope, a cardioscope, and an arthroscope. A length, a diameter and/or a rigidity/bendability of the tube may be chosen accordingly.

The tube may for example be an insertion tube for use in an endoscope or a part of an insertion tube for use in an endoscope. In one example, the tube is a hypotube for use in an endoscope. The hypotube may for example be configured to be surrounded or enclosed by a tubular sleeve or cover to form an insertion tube. The hypotube may for example form a frame or skeleton of the insertion tube that is configured to provide dimensional stability to the insertion tube.

The present disclosure further provides an endoscope including a tube according to any one of the examples described herein. The tube may for example be an insertion tube of the endoscope or a part thereof, in particular a hypotube. The endoscope may further include an interface/control portion that is configured to be coupled to the tube.

### LIST OF FIGURES

In the following, a detailed description of the present disclosure and examples thereof is given with reference to the figures. The figures show schematic illustrations of
Fig. 1: an endoscope according to an example of the present disclosure;
Fig. 2a: a joint with a joint head and a joint socket in accordance with an example of the present disclosure;
Fig. 2b: the joint of Fig. 2a at a default articulation angle;
Fig. 2c: the joint of Fig. 2a at a first target articulation angle;
Fig. 2d: the joint of Fig. 2a at a second target articulation angle;
Fig. 3a: a pair of tube elements having a joint head and a joint socket according to an example of the present disclosure in side view;
Fig. 3b: the joint head and joint socket of the tube elements of Fig. 3a in a close-up view;
Fig. 3c: one of the tube elements of Fig. 3a in front view;
Fig. 3d: one of the tube elements of Fig. 3a in back view;
Fig. 3e: one of the tube elements of Fig. 3a in a perspective view;
Fig. 4a: an active bending section of a tube for use in an endoscope according to an example of the present disclosure in a first side view;
Fig. 4b: the active bending section of Fig. 4a in a second side view;
Fig. 5a: a pair of tube elements having a joint head and a joint socket according to another example of the present disclosure in side view;
Fig. 5b: one of the tube elements of Fig. 5a in a perspective view;
Fig. 5c: one of the tube elements of Fig. 5a in front view;
Fig. 5d: one of the tube elements of Fig. 5a in back view;
Fig. 5e: one of the tube elements of Fig. 5a in another perspective view;
Fig. 6a: an active bending section of a tube for use in an endoscope according to another example of the present disclosure in a first side view; and
Fig. 6b: the active bending section of Fig. 6a in a second side view.

### DESCRIPTION OF EXAMPLES

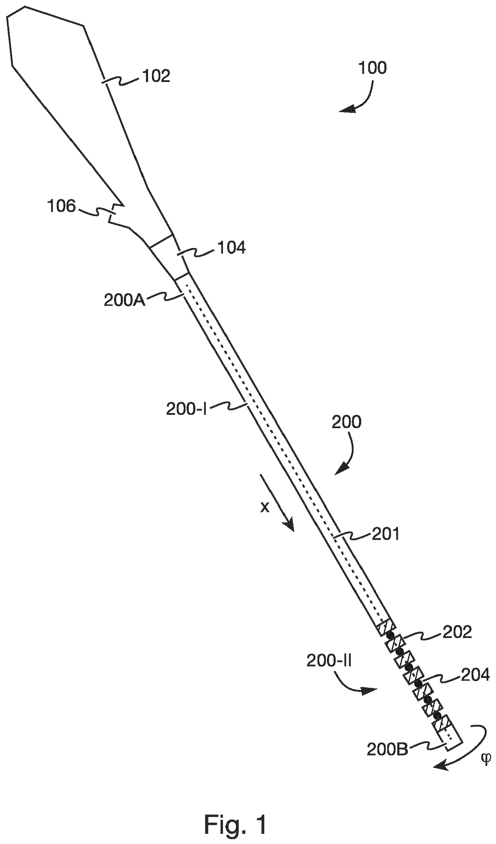

Fig. 1 shows a schematic illustration (not to scale) of an endoscope 100 according to an example of the present disclosure. The endoscope 100 includes an interface/control portion 102 with a connector 104 and one or more ports 106.

The connector 104 is configured to connect or attach a tube 200 to the interface/control portion 102, wherein the tube 200 may be connected or attached removably in some examples. The tube 200 extends along a center line 201 of the tube 200 from a proximal end 200A to a distal end 200B, wherein the proximal end 200A is adjacent to the interface/control portion 102 (e.g. connected or attached thereto via the connector 104) and the distal end 200B is facing away from the interface/control portion 102. A direction X along the center line 201 from the proximal end 200A to the distal end 200B may be referred to as a longitudinal, axial or X direction in the following. A direction ϕ along a circumference of the tube (e.g. parallel to a surface of the tube and perpendicular to the longitudinal direction X) may be referred to as a circumferential, azimuthal or ϕ direction in the following.

The one or more ports 106 may for example include one or more fluid ports that are in fluid communication with an interior of the tube 200, for example with a channel (not shown) formed by or arranged in the tube 200, e.g. via the connector 104. Additionally or alternatively, the one or more ports 106 may include one or more light guide ports, wherein each of the light guide ports may for example be configured to receive a light guide (not shown) that is to be arranged within the tube 200 and/or to provide coupling to a light guide (not shown) that is arranged within the tube 200, for example in a channel formed by or arranged in the tube 200. The interface/control portion 102 may further include one or more electrical contacts (not shown), e.g. to provide an electrical connection to the interior of the tube 200, to a distal end 200B of the tube 200 and/or to a distal tip (not shown) of the endoscope 100, which may e.g. be arranged at or connected to the distal end 200B of the tube 200. The interface/control portion 106 may also include means for actuating a distal section 200-II of the tube 200, for example one or more control knobs (not shown), which may e.g. be configured to apply tension to one or more control wires (not shown) coupled to the distal section 200-II, e.g. via one or more attachment point at the distal end 200B.

The interface/control portion 102 may be embodied as a single unit as shown in Fig. 1 or as two or more separate units, for example a control portion and an interface portion, wherein the control portion may e.g. be connected to the proximal end 200A of the tube 200 via the connector 104 and the interface portion may e.g. be connected to the control portion via a flexible tube or an umbilical cord.

The tube 200 may be a tube for use in an endoscope according to any one of the examples described herein. In the example of Fig. 1, the tube 200 includes a first or proximal section 200-I and a second or distal section 200-II. The proximal section 200-I may for example be or include one or more passive bending sections (not shown) that are configured to be bent or deformed when applying an external force. The passive bending sections may for example be made of (e.g. include) a flexible material. Additionally or alternatively, the passive bending sections may be made of a rigid or semi-rigid material such as metal (e.g. stainless steel) and may for example include a plurality of bendable segments, in each of which one or more openings may be provided in a wall of the tube 200 to increase a flexibility of the tube 200 in the respective bendable segment. In some examples, the proximal section 200-I may also include a rigid section (not shown), either in addition to or instead of the one or more passive bending section, e.g. at the proximal end 200A adjacent to the connector 104. A length and a diameter of the tube may be chosen to be suitable for the type of endoscope that the tube 200 is to be used with. The tube 200 may for example have a length between 20 cm and 200 cm, in some examples between 50 cm and 150 cm. An outer diameter of the tube 200 may for example be between 1 mm and 20 mm, in some examples between 2 mm and 10 mm.

In the example of Fig. 1, the distal section 200-II is or includes a bending section, in particular an active bending section and may thus also be referred to as the active bending section 200-II. The active bending section 200-II includes a plurality of elements 202 (also referred to as tube elements 202 in the following) arranged along the center line 201 of the tube 200. The tube element 202 are pivotably connected to each other by a plurality of joints 204 so as to allow for bending the tube 200, in particular the active bending section 200-II, with respect to a default configuration of the tube 200. The default configuration may for example be a straight configuration as shown in Fig. 1, in which the tube 200 is not bent and the center line 201 is a straight line. The default configuration may for example be the configuration that the tube 200 ideally (e.g. without any external perturbation) assumes when no additional tension is applied to the control wires, e.g. such that the control wires create a uniform (non-directional) compressive tension in the longitudinal direction. In some examples, the active bending section 200-II may be actuated or controlled actively, e.g. using corresponding means for actuating the active bending section 200-II such as control wires. In other examples, the section 200-II may be a passive bending section, wherein the passive bending section may for example bend or deform under external forces that cause the joints to pivot.

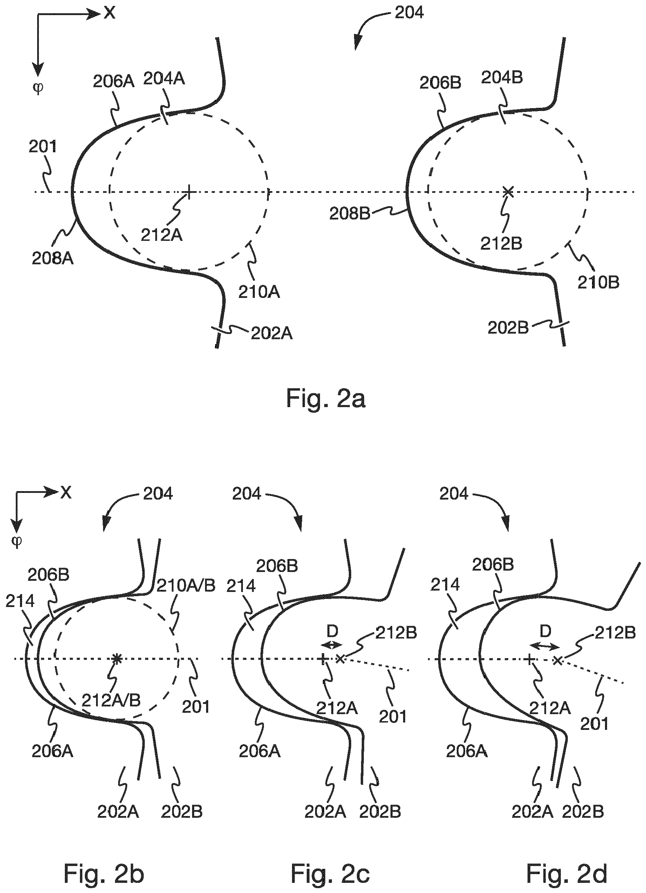

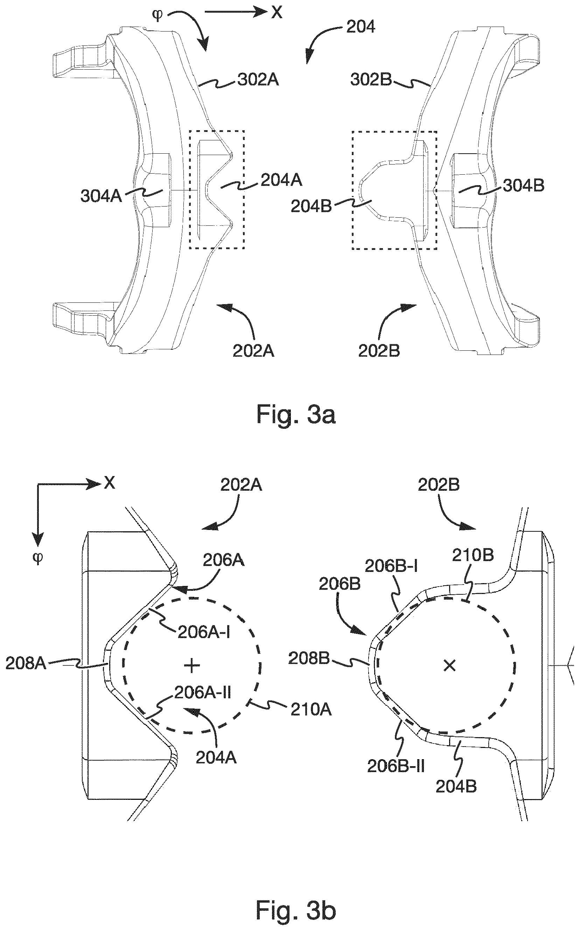

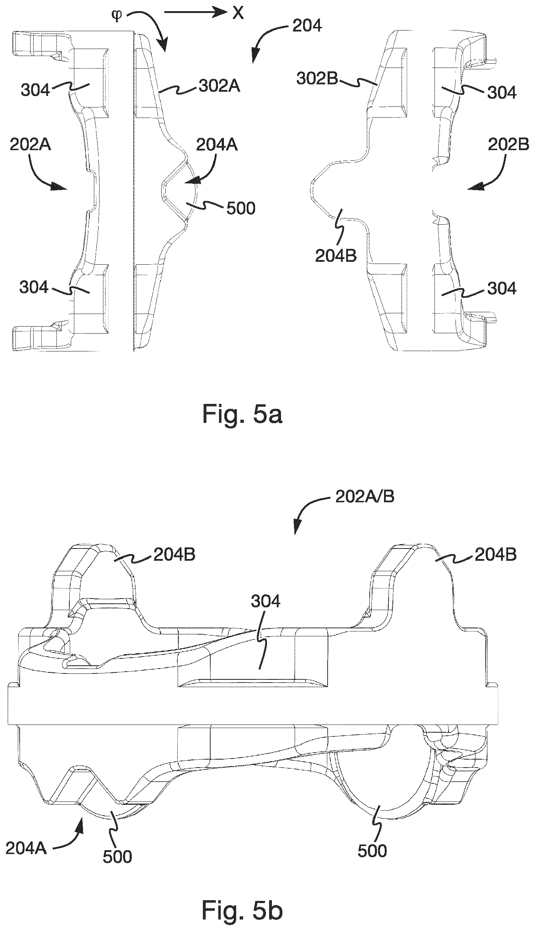

Each of the joints 204 is configured such that when the elements 202 connected by the respective joint 204 are pivoted with respect to each other from a default articulation angle of the joint 204 associated with the default configuration (e.g. 0° in the example of Fig. 1) to a target articulation angle different from the default articulation angle (i.e. a non-zero angle in the example of Fig. 1), the joint 204 extends or expands along the longitudinal direction to slightly increase a length of the tube 200 along the longitudinal direction, e.g. as detailed below with reference to Figs. 2a-2d. Each of the joints 204 may for example be embodied similar to the joint of Figs. 2a-2d, the joint of Figs. 3a, 3b or the joint of Fig. 5a. In the default configuration, the tube 200 (e.g. the center line 201 thereof) may have its smallest longitudinal extent, i.e. the smallest length in the longitudinal direction. When a uniform tension is applied in the longitudinal direction, the tube 200 may tend to reduce or minimize its length and may assume the default configuration, i.e. straighten in the example of Fig. 1.

Fig. 2a to 2d show schematic illustrations (not to scale) of a joint 204 of a tube for use in an endoscope in accordance with an example of the present disclosure. The joint 204 may for example be used in the tube 200 of Fig. 1 to pivotably connect two elements 202 to each other. In the following, the tube 200 will be used as a non-limiting example for illustration purposes. The direction of view in Figs. 2a to 2d may be parallel to an articulation axis of the joint 204 and may for example correspond to the radial direction of the tube 200.

The joint 204 includes a joint head 204B on a respective first element 202B (e.g. a distal element of the joint 204 that is closer to the distal end 200B of the tube 200) and a joint socket 204A on a respective second element 202A (e.g. a proximal element of the joint 204 that is closer to the proximal end 200A of the tube 200). The joint socket 204A is configured to receive the joint head 204B as illustrated in Figs. 2a to 2d. In Fig. 2a, the joint 204 is depicted with the joint head 204B removed from the joint socket 204A, e.g. when assembling the tube 200. In Figs. 2b to 2d, the joint 204 is depicted with the joint head 204B arranged in the joint socket 204A. In Fig. 2b, the joint 204 is at its default articulation angle, e.g. 0° in this example corresponding to a straight center line 201, whereas in Figs. 2c and 2d, the joint 204 is at a first and second target articulation angle, respectively, different from the default articulation angle, e.g. 10° in the example of Fig. 2c and 20° in the example of Fig. 2d. Accordingly, the center line 201 is bent or kinked in Figs. 2c and 2d.

The joint head 204B includes a first contact surface 206B that is configured to slidably engage a second contact surface 206A on the joint socket 204A when the joint head 204B is received in the joint socket 204B. The joint socket 204A and the joint head 204B, in particular the first and second contact surfaces 206A, 206B, are shaped such that, when the first element 202B is pivoted with respect to the second element 202A away from the default articulation angle, e.g. going from the default configuration of Fig. 2b to the configuration of Fig. 2c, the first contact surface 206B engages the second contact surface 206A to cause the joint head 204B to be pushed outwards out of the joint socket 204A. Thereby, the joint head 204B - as seen from the joint socket 204A - is displaced by a displacement D relative to its position at the default articulation angle in Fig. 2b such that the joint 204 extends or expands slightly along the longitudinal direction. This may lead to the length of the tube 200 increasing, e.g. by the displacement D. The joint head 204B and the joint socket 204A are configured such that the displacement D is greater than zero at any target articulation angle (e.g. any non-zero articulation angle), i.e. the joint 204 (and thus the tube 200) has its smallest longitudinal extent at the default articulation angle.

In the example of Figs. 2a-2d, both the joint socket 204A and the joint head 204B have a non-circular cross-section perpendicular to an articulation axis of the joint 204 (e.g. perpendicular to the radial direction of the tube 200). In particular, the first contact surface 206B of the joint head 204B includes a protruding portion 208B that protrudes from an inscribed circle 210B of the first contact surface 206B. The second contact surface 206A of the joint head 204A includes a recessed portion 208A that is recessed from an inscribed circle 210A of the second contact surface 206A. In the example of Figs. 2a-2d, the inscribed circles 210A, 210B are perpendicular to said articulation axis of the joint 204 and defined such that portions of the respective contact surface 208A, 208B that are in contact with the other contact surface 208B, 208A at the default articulation angle are tangential to the inscribed circle 210A, 210B as illustrated in Fig. 2b. At the default articulation angle, a center 212A of the inscribed circle 210A of the second contact surface 206A may be aligned with a center 212B of the inscribed circle 210B of the second contact surface 206B. The centers 212A, 212B may for example lie on the articulation axis of the joint 204 at the default articulation angle. The inscribed circles 210A, 210B may for example be the largest circles that can be inscribed to the first and second contact surfaces 206A, 206B, respectively, and are centered on the articulation axis of the joint 204 at the default articulation angle.

At the default articulation angle, the protruding portion 208B faces the recessed portion 208A and is separated from the recessed portion 208A by a gap 214. The gap 214 may for example have a width along the longitudinal direction of between 10 µm and 100 µm. When the first element 202B is pivoted away from the default articulation angle, the protruding portion 208B comes in contact with the second contact surface 206A, e.g. with a lateral portion thereof that is adjacent to the recessed portion 206A. This causes the joint head 204B to be pushed outwards out of the joint socket 204A such that the joint head 204B is displaced by a displacement D along the center line 201. The displacement D may for example be the distance by which the center 212B of the inscribed circle 210B of the first contact surface 206B is displaced from the center 212A of the inscribed circle 210A of the second contact surface 206A. In some examples, the displacement D may increase monotonically with the articulation angle, e.g. with a modulus of the difference between the target articulation angle and the default articulation angle. The displacement D may increase monotonically over a certain range of articulation angles (e.g. up to an articulation angle of at least 20°, in some examples of at least 30°) and/or over the entire operable range of the joint 204 (e.g. up to a maximum articulation angle of the joint 204).

The motion of the joint head 204B when pivoting the first element 202B corresponds to a combined rotation and translation. The motion leads to a rotation or tilting of the first element 202B and thus of the corresponding portion of the center line 201 by the articulation angle as well as to the displacement of the joint head 204B. Accordingly, the articulation axis of the joint 204 may shift when pivoting the first element 202B and may be at a different position at each articulation angle. The articulation axis may not tilt, but may always remain parallel to its orientation at the default angle (i.e. may be shifted or translated by a parallel offset), e.g. parallel to the radial direction.

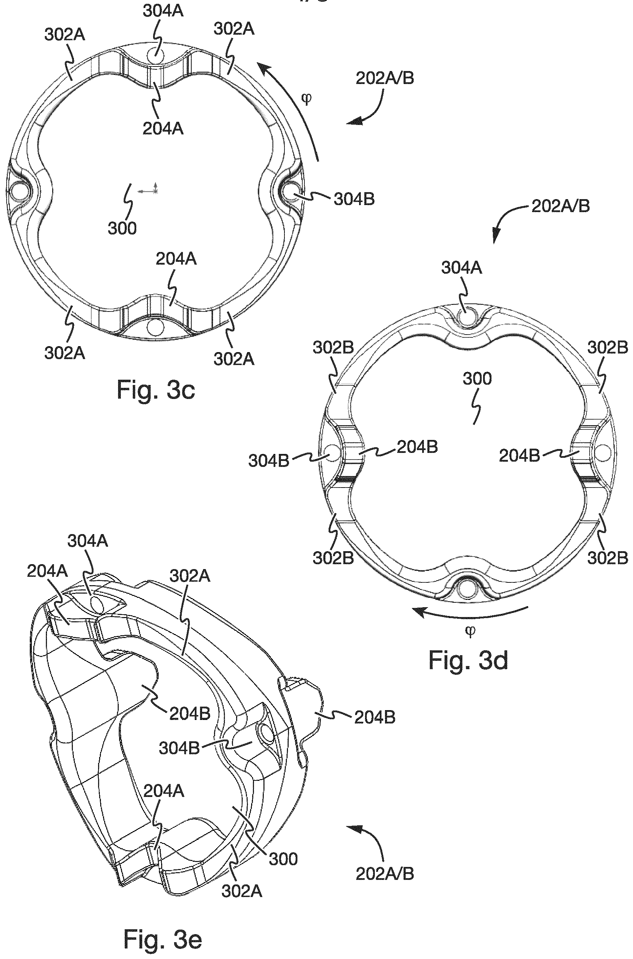

Fig. 3a depicts a schematic illustration of a pair of tube elements 202A, 202B having a joint socket 204A and a joint head 204B, respectively, according to an example of the present disclosure in side view. The tube elements 202A, 202B may for example be used in the tube 200 of Fig. 1, which will be used as a non-limiting example for illustration purposes in the following. Fig. 3b shows a close-up view of the joint socket 204A and the joint head 204B corresponding to the dotted rectangles in Fig. 3a. In Figs. 3c and 3d, one of the tube elements 202A, 202B is shown in front view (e.g. along the longitudinal direction of the tube 200 facing the proximal end 200A) and back view (e.g. along the longitudinal direction of the tube 200 facing the distal end 200B), respectively. Fig. 3e depicts one of the tube elements 202A, 202B in a perspective view.

The tube elements 202A, 202B may for example made of (e.g. include, i.e. including, but not limited to) a semi-rigid or rigid material, in particular a metal such as stainless steel, titanium or a nickel-titanium alloy such as Nitionol. In some examples, the tube elements 202A, 202B may consist of (i.e. including nothing else but) a semi-rigid or rigid material, in particular a metal such as stainless steel, titanium or a nickel-titanium alloy such as Nitionol.

The joint socket 204A is configured to receive the joint head 204B to form an articulatedjoint 204. Similar to the joint of Figs. 2a to 2d, the joint head 204B includes a first contact surface 206B with a protruding portion 208B that protrudes from an inscribed circle 210B of the first contact surface 206B. The joint socket 204A includes a second contact surface 206B that is configured to slidably engage the first contact surface 206A to permit articulation of the joint 204. The second contact surface 206A includes a recessed portion 208A that is recessed from an inscribed circle 210A of the second contact surface 206A.

In the example of Figs. 3a, 3b, the first contact surface 206B has a flat-topped triangular or V-shape (e.g. a flat-topped triangular or V-shaped contour or outline in a plane perpendicular to an articulation axis of the joint 204). The first contact surface 206B includes a pair of straight portions 206B-I, 206B-II arranged on opposite sides of the protruding portion 208B. The second contact surface 206A has a corresponding flat-topped triangular or V-shape and also includes a pair of straight portions 206A-I, 206A-II arranged on opposite sides of the recessed portion 208A. Each of the straight portions 206A-I, 206A-II, 206B-I, 206B-II is tangential to the inscribed circle 210A, 210B of the respective contact surface 206A, 206B. The inscribed circle 210A, 210B may for example extend through the centers of the respective straight portions 206A-I, 206A-II, 206B-I, 206B-II as illustrated in Fig. 3b. When the joint head 204B is received in the joint socket 204A at a default articulation angle (e.g. 0° as in the configuration shown in Figs. 3a, 3b), the straight portions 206A-I, 206A-II, 206B-I, 206B-II may be in contact with each other. This may provide a robust and well-defined contact between the joint socket 204B and the joint head 204A.

The first and second elements 202A, 202B each include a first and second angled end face 302A, 302B, respectively. The end faces 302A, 302B face each other and are configured to come in contact with each other when the joint 204 reaches its maximum articulation angle, thereby preventing further articulation of the joint 204. The maximum articulation angle may for example correspond to the angle between the end faces 302A, 302B when the joint is at the default articulation angle and may for example be between 5° and 75°, in some examples between 25° and 55°, in one example between 35° and 45°.

The tube elements 202A, 202B are annular elements that extend around (e.g. surround or enclose) a channel opening 300 in the circumferential direction. In some examples, the tube elements 202A, 202B may be identical elements, wherein the second element 202A may for example be rotated with respect to the first element 202B by 90° along the circumferential direction. Each of the tube elements 202A, 202B may include a pair of joint heads 204B on a first end face (e.g. on a proximal end face) and a pair of joint sockets 204A on a second end face opposite to the first end face (e.g. on a distal end face). The joint sockets 204A may be arranged on opposite sides of the tube elements along the circumferential direction, e.g. rotated by 180° with respect to each other along the circumferential direction. The joint heads 204B may also be arranged on opposite sides of the tube elements along the circumferential direction, e.g. rotated by 180° with respect to each other along the circumferential direction, for example such that the joint sockets 204A and joint heads 204B are spaced equidistantly (e.g. spaced apart by 90°) along the circumferential direction as shown in Figs. 3c to 3e.

Each of the tube elements 202A, 202B further includes four guiding elements 304A, 304B, each of which is configured to guide a control wire for actuating the active bending section 200-II of the tube 200. Each of the guiding elements 304A, 304B is arranged at an outer circumference of the respective tube element 202A, 202B and includes a groove and a hole in which the respective control wire can be arranged. A first pair of guiding elements 304A is arranged adjacent to a respective one of the joint sockets 204A, e.g. aligned with the respective joint socket 204A along the circumferential direction as shown in Figs. 3c to 3e. A second pair of guiding elements is arranged adjacent to a respective one of the joint heads 204B, e.g. aligned with the respective joint head 204B along the circumferential direction as shown in Figs. 3c to 3e.

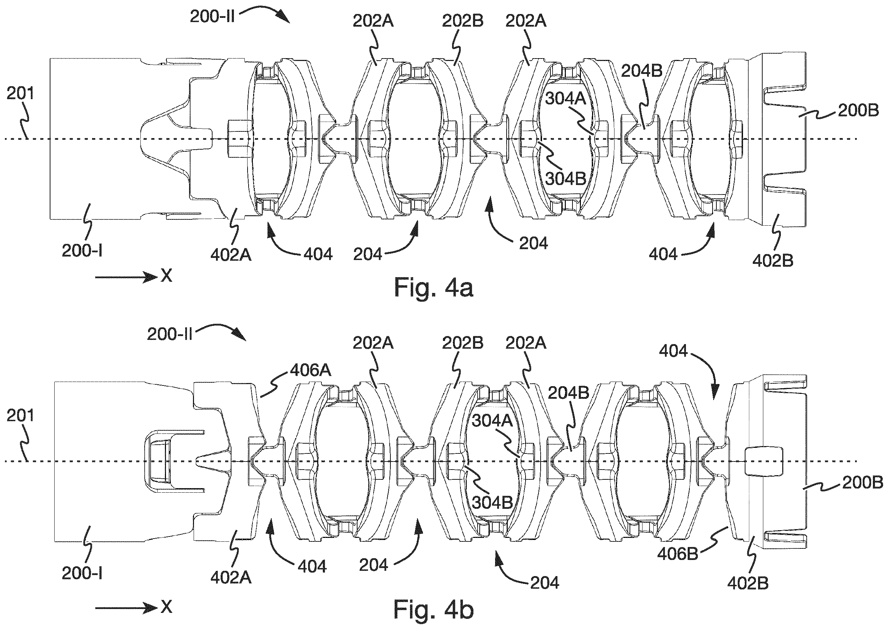

Figs. 4a, 4b depict a schematic illustration of an active bending section 200-II of a tube for use in an endoscope according to an example of the present disclosure. Fig. 4a shows the active bending section 200-II in a first side view and Fig. 4b shows the active bending section 200-II in a second side view, which may e.g. be orthogonal to the direction of view of Fig. 4a.

The active bending section 200-II may for example be the distal section 200-II of the tube 200 or may form a part thereof. The tube 200 is therefore used as a non-limiting example for illustration purposes in the following. The active bending section 200-II connects the proximal section 200-I of the tube 200 with a tip of the tube 200 at the distal end 200B, wherein the tip of the tube 200 may for example be formed by an outermost distal element 402B of the active bending section 200-II. The active bending section 200-II may allow for actively navigating the tip of the tube 200, e.g. using control means such as control knobs provided in the interface/control portion 102.

For this, the active bending section 200-II includes a plurality of elements 202A, 202B, 402A, 402B, some or all of which (e.g. all of the elements 202A, 202B arranged between the outermost elements 402A, 402B) may for example be embodied similar to the tube elements of Figs. 3a to 3e. The active bending section 200-II may for example include between 3 and 50 elements, in some examples between 5 and 20 elements, e.g. 8 elements as in the example of Figs. 4a, 4b. The elements 202A, 202B are arranged in a chain, wherein each of the elements 202A, 202B (with the exception of the outermost elements 402A, 402B) is pivotably connected to a respective proximal element by a pair of proximal joints 204 and to a respective distal element by a pair of distal joints 204 with each of the joints 204 being formed by a joint socket 204A and a joint head 204B as detailed above with reference to Figs. 2a to 2d, 3a to 3e.

In the example of Figs. 4a, 4b, the joints 204 are arranged in two articulation planes, wherein every other pair of joints along the chain (e.g. the first, third, fifth, ... pairs of joints along the chain, which are visible in Fig. 4b and also referred to as the first set of joints) is arranged in a first articulation plane and the remaining joints (e.g. the second, fourth,... pairs of joints, which are visible in Fig. 4a and also referred to as the second set of joints) are arranged in a second articulation plane that is orthogonal to the first articulation plane. In other words, on each element 202A, 202B, the pair of proximal joints 204 is displaced with respect to the pair of distal joints 204 by 90° along the circumferential direction. Each set of joints is configured to permit articulation of the active bending section 200-II in a direction or plane perpendicular to the respective articulation plane.

The guiding elements 304A, 304B on the tube elements 202A, 202B are each aligned with one of the joints 204 as detailed above with reference to Figs. 3c to 3e such that the control wires, when arranged in the guiding elements 304A, 304B, extend in two wire planes that are aligned with the articulation planes, thereby permitting actuation of the set of joints associated with the articulation plane that is perpendicular to the wire plane that the respective wire is arranged in (and thus causing the active bending section 200-II to be bent in the wire plane that the respective wire plane is arranged in).

With the exception of the outermost elements 402A, 402B, all of the tube elements 202A, 202B may be identical elements with every other element being rotated by 90° along the circumferential direction (such that the joint sockets 204A are aligned with the joint heads 204B on the adjacent element to form the joints 204). The outermost proximal element 402A may be configured to be connected or attached to the proximal section 200-I, whereas the outermost distal element 402B may form the distal tip of the tube 200 and may for example be configured to accommodate a camera and/or one or more medical tools. The outermost distal element 402B may also include one or more attachment points at which a respective control wire can be connected or attached to the outermost distal element 402B.

The outermost elements 402A, 402B further differ from the other elements 202A in that their end faces 406A, 406B are angled at a different angle with respect to a plane perpendicular to the center line 201 of the tube 200. In particular, the end faces 406A, 406B extend at a smaller angle with respect to said plane than the end faces of the other element 202A. Accordingly, the angle between each of the end faces 406A, 406B and the opposing end face of the respective adjacent element 202A, 202B (and thus a maximum articulation angle of the outermost joints 404) is smaller than the angle between opposing end faces of the elements 202A, 202B (and thus a maximum articulation angle of the joints 204). For example, the angle between opposing end faces of the elements 202A, 202B maybe between 35° and 45°, e.g. 40°. The angle between each of the end faces 406A, 406B and the opposing end face of the respective adjacent element 202A, 202B may for example be between 25° and 35°, e.g. 30° in one example or 2/3 of the angle between opposing end faces of the elements 202A, 202B in another example. In other examples, the angle between each of the end faces 406A, 406B and the opposing end face of the respective adjacent element 202A, 202B may be between 5° and 20°, e.g. 15° in one example or 1/3 of the angle between opposing end faces of the elements 202A, 202B in another example.

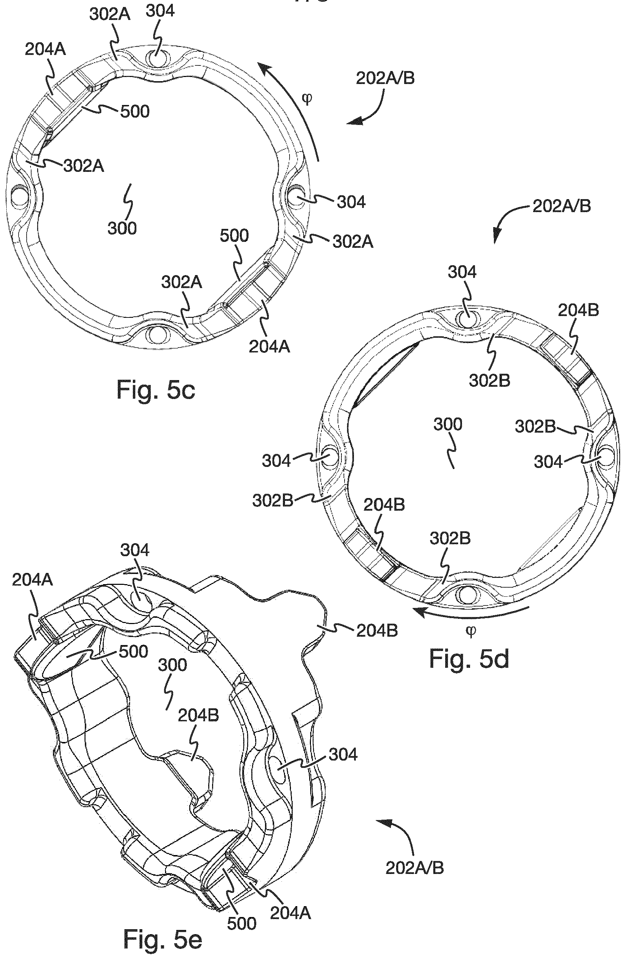

Fig. 5a depicts a schematic illustration of a pair of tube elements 202A, 202B having a joint socket 204A and a joint head 204B, respectively, according to another example of the present disclosure in side view. The tube elements 202A, 202B may for example be used in the tube 200 of Fig. 1, which will be used as a non-limiting example for illustration purposes in the following. Figs. 5b and 5e depict one of the tube elements 202A, 202B in two different perspective views. In Figs. 5c and 5d, one of the tube elements 202A, 202B is shown in front view (e.g. along the longitudinal direction of the tube 200 facing the proximal end 200A) and back view (e.g. along the longitudinal direction of the tube 200 facing the distal end 200B), respectively.

The tube elements 202A, 202B are similar to the tube elements of Figs. 3a to 3e. In particular, the tube elements 202A, 202B are also annular elements including a pair of flat-topped triangular or V-shaped joint heads 204B on a first (e.g. proximal) end face as well as a pair of corresponding flat-topped triangular or V-shaped joint sockets 204A on a second (e.g. distal) end face, for example as illustrated in Fig. 3b.

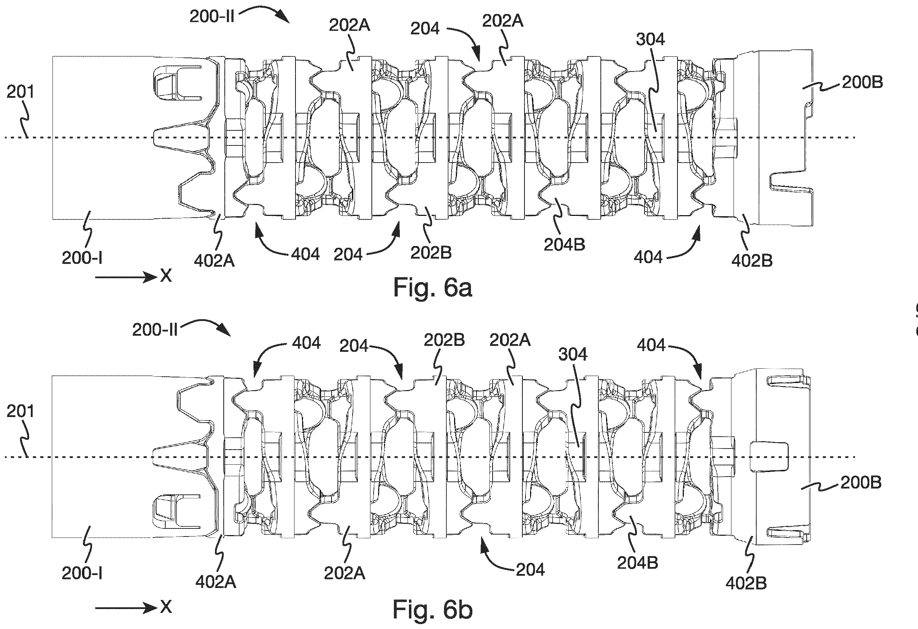

The tube elements 202A, 202B differ from the tube elements of Figs. 3a to 3e in the arrangement of the guiding elements 304 with respect to the joint heads and sockets 204A, 204B. In the example of Figs. 5a to 5e, the guiding elements 304 are displaced with respect to the joint heads and sockets 204A, 204B such that each of the guiding elements 304 is arranged between a respective joint head 204B and a respective joint 204A, e.g. halfway between the joint head 204 and the joint socket 204A (displaced by 45° along the circumferential direction from each of the joint head 204B and joint socket 204A). This may for example allow for forming a chain of tube elements in which the articulation planes of the joints 204 and the wire planes of the control wires are not aligned, e.g. as described below with reference to Figs. 6a, 6b.

The tube elements 202A, 202B further include lateral mechanical stops 500 that are configured to limit a movement of the joint heads 204B relative to the respective joint socket 204A in a direction parallel to an articulation axis of the joint (e.g. in the radial direction). In the example of Figs 5a to 5e, the lateral mechanical stops 500 are sidewalls of the joint sockets 204A arranged at an inner circumference of the respective tube element 202A, 202B. The sidewalls may for example extend over the entire depth of the respective joint socket along the longitudinal direction, e.g. as shown in Fig. 5a. In some examples, the sidewalls may include a tip portion, e.g. a circular tip portion, that is configured to engage a corresponding contact surface on an adjacent tube element 204A/B.

Figs. 6a, 6b depict a schematic illustration of an active bending section 200-II of a tube for use in an endoscope according to another example of the present disclosure. Fig. 6a shows the active bending section 200-II in a first side view and Fig. 6b shows the active bending section 200-II in a second side view, which may e.g. be orthogonal to the direction of view of Fig. 6a.

The active bending section 200-II is similar to the active bending section of Figs. 4a, 4b and also includes a chain of tube elements 202A, 202B, 402A, 402B that are pivotably connected to each other by a plurality of articulated joints 204, 404 formed by joint heads 204A and joint sockets 204B on the tube elements 202A, 202B, 402A, 402B. With the exception of the outermost elements 402A, 402B, each of the elements 202A, 202B may for example be embodied similar to the tube elements of Figs. 5a to 5e. The elements 202A, 202B are arranged such that adjacent elements are rotated by 90° with respect to each other along the circumferential direction.

As in the example of Figs. 4a, 4b, the joints 204 are arranged in two articulation planes. Every other pair of joints along the chain (e.g. the first, third, fifth, ... pairs of joints, also referred to as the first set of joints) is arranged in a first articulation plane (tilted by +45° with respect to the direction of view of Fig. 6a and by -45° with respect to the direction of view of Fig.6b). The remaining joints (e.g. the second, fourth,... pairs of joints, also referred to as the second set of joints) are arranged in a second articulation plane that is orthogonal to the first articulation plane (tilted by -45° with respect to the direction of view of Fig. 6a and by +45° with respect to the direction of view of Fig. 6b). Each set of joints is configured to permit articulation of the active bending section 200-II in a direction or plane perpendicular to the respective articulation plane.

As detailed above with reference to Figs. 5a to 5e, the guiding elements 304 on the tube elements 202A, 202B are displaced from adjacent joint sockets 204A and joint heads 204B on the respective tube element 202A/B (and thus from adjacent joints) by 45° along the circumferential direction. Accordingly, the control wires, when arranged in the guiding elements 304 extend in two wire planes that are tilted or rotated by 45° with respect to each of the articulation planes. For example, a first pair of guiding elements 304 on each of the elements 202A, 202B is arranged in a first wire plane parallel to the direction of view of Fig. 6a and a second pair of guiding elements on each of the elements 202A, 202B is arranged in a second wire plane perpendicular to the direction of view of Fig. 6a (and thus parallel to the direction of view of Fig. 6b). When one or both control wires in a given wire plane are actuated (e.g. by pulling or releasing the respective control wire to adjust a longitudinal tension generated by the respective control wire via the attachment point on the outermost distal element 402B), both the first set of joints and the second set of joints are actuated simultaneously, causing the active bending section 200-II to be bent in the respective wire plane. As compared to an arrangement as in Figs. 4a, 4b, the articulation angle of each actuated joint at a given bending radius of the active bending section 200-II may for example be about half as large.

The examples of the present disclosure disclosed herein only constitute specific examples for illustration purposes. The present invention can be implemented in various ways and with many modifications without altering the underlying basic properties. Therefore, the present invention is only defined by the claims as stated below.

## Claims

1. A tube (200) for use in an endoscope (100), wherein the tube (200) comprises a plurality of elements (202, 402A, 402B) that are arranged along a center line (201) of said tube (200) and pivotably connected to each other by a plurality of joints (204, 404), said joints (202, 404) allowing for bending the tube (200) with respect to a default configuration of the tube (200), wherein each of the joints (204, 404) comprises:
a joint head (204B) on a respective first element (202B, 402B) of the plurality of elements (202, 402A, 402B); and
a joint socket (204A) on a respective second element (202A, 402A) of the plurality of elements (202, 402A, 402B), the joint socket (204A) being configured to receive the joint head (204B),
wherein the joint socket (204A) and the joint head (204B) are shaped such that, when the first element (202B, 402B) is pivoted with respect to the second element (202A, 402A) from a default articulation angle of the joint (204) associated with said default configuration to a target articulation angle:
a first contact surface (206B) on the joint head (204B) engages a second contact surface (206A) on the joint socket (204A) to cause the joint head (204B) to be pushed outwards out of the joint socket (204A) such that a displacement (D) of the joint head (204B) along said center line (201) of the tube (200) relative to its position at the default articulation angle is greater than zero for any target articulation angle.

2. The tube (200) of claim 1, wherein:
the first contact surface (206B) comprises a protruding portion (208B) that protrudes from an inscribed circle (210B) of the first contact surface (206B) and the second contact surface (206A) comprises a recessed portion (208A) that is recessed from an inscribed circle (210A) of the second contact surface (206A);
the protruding portion (208B) of the first contact surface (206B) faces the recessed portion (208A) of the second contact surface (206A) when the first element (202B, 402B) is at the default articulation angle; and
when the first element (202B, 402B) is pivoted from the default articulation angle to the target articulation angle, the protruding portion (208B) engages the second contact surface (206A) to cause the joint head (204B) to be pushed outwards out of the joint socket (204A).

3. The tube (200) of claim 1 or 2, wherein the first contact surface (206B) comprises a first straight portion (206B-I) and a second straight portion (206B-II) extending at an angle to the first straight portion (206B-I), the first and second straight portions (206B-I, 206B-II) being arranged such that, when the first element (202B, 402B) is at the default articulation angle, the first and second straight portions (206B-I, 206B-II) each are in contact with a respective straight portion (206A-I, 206A-II) of the second contact surface (206A).

4. The tube (200) of claim 2 and 3, wherein the first and second straight portions (206BI, 206B-II) of the first contact surface (206B) are tangential to the inscribed circle (210B) of the first contact surface (206B) and the protruding portion (208B) of the first contact surface (206B) is arranged between the first and second straight portions (206B-1, 206B-II).

5. The tube (200) of any one of the preceding claims, wherein the joint socket (204A) and the joint head (204B) are shaped such that the displacement of the joint head (204B) along said center line (201) of the tube (200) relative to its position at the default articulation angle increases monotonically when the first element (202B, 402B) is pivoted from the default articulation angle to a target articulation angle that differs from the default articulation angle by no less than 10°, preferably by no less than 20°.

6. The tube (200) of any one of the preceding claims, wherein the joint socket (204A) and the joint head (204B) are shaped such that a tip of the joint head (204B) is separated from the joint socket (204A) by a gap (214) when the first element (202B, 402B) is at the default articulation angle.

7. The tube (200) of any one of the preceding claims, wherein:
the joint socket (204A) is configured such that the joint head (204B) can be removed from the joint socket (204A) along the center line (201) of the tube (200); and/or
the joint socket (204A) and/or the joint head (204B) comprises a lateral mechanical stop (500) configured to limit a movement of the joint head (204B) relative to the joint socket (204A) in a direction parallel to an articulation axis of the joint (204, 404).

8. The tube (200) of any one of the preceding claims, wherein one or more joints (204, 404) of the plurality of joints (204, 404) have a maximum articulation angle that is different from a maximum articulation angle of the other joints (204, 404) of the plurality of joints (204, 404).

9. The tube (200) of claim 8, wherein the elements (202, 402A, 402B) are arranged in a chain and an outermost joint (404) at one or both ends of the chain has a smaller maximum articulation angle than the other joints (204) in the chain, in particular wherein the maximum articulation angle of the outermost joint (404) at one or both ends of the chain is between 10% and 70% of the maximum articulation angle of the other joints (204) in the chain.

10. The tube (200) of claim 9, wherein:
for each of the joints (204, 404), the first element (202B, 402B) comprises a first angled end face (302B) and the second element (202A, 402A) comprises a second angled end face (302A) facing the first angled end face (302B), the first and second end faces (302B, 302A) being configured to come in contact with each other when the joint (204, 404) reaches its maximum articulation angle to prevent further articulation of the joint (204, 404); and
an angle between the first and second end faces (302B, 302A) associated with the outermost joint (404) at one or both ends of the chain is smaller than an angle between the first and second end faces (302B, 302A) associated with the other joints (204) in the chain when the respective joints (204, 404) are at the default articulation angle.

11. The tube (200) of any one of the preceding claims, wherein some or all of the elements (202, 402A, 402B) comprise a guiding element (304, 304A, 304B) configured to guide a control wire for actuating the plurality of joints (204, 404).

12. The tube (200) of claim 11, wherein:
the guiding element (304, 304A, 304B) is arranged at an outer circumference of the respective element (202, 402A, 402B); and/or
the guiding element (304A, 304B) is configured to guide the control wire such that the control wire is arranged adjacent to an inner or outer sideface of a joint head (206B) on the respective element (202B) or on an adjacent element (202B).

13. The tube (200) of claim 11 or 12, wherein at least some of the elements (202A, 202B) each:
are pivotably connected to a respective first adjacent element (202B, 202A) by a respective first pair of joints (204) arranged on opposite sides of the tube (200) and to a respective second adjacent element (202B, 202A) by a respective second pair of joints (204) arranged on opposite sides of the tube, the first and second pairs of joints (204) being displaced with respect to each other by between 75° and 105° along a circumferential direction (ϕ) of the tube (200); and
comprise four guiding elements (304) arranged along a circumference of the respective element (202A, 202B), each of said four guiding elements (304) being displaced by between 35° and 55° from adjacent joints (204) along the circumferential direction (ϕ) of the tube (200).

14. The tube (200) of any one of the preceding claims, wherein each of the elements (202, 402A, 402B) extends around a respective channel opening (300) and a diameter of said channel opening (300) varies by less than 25%, preferably by less than 15% along the circumference of the respective element (202, 402A, 402B).

15. The tube (200, 700) of any one of the preceding claims, wherein the tube (200) is an insertion tube or a hypotube for use in said endoscope (100).
